# EUROPÄISCHE PATENTSCHRIFT

(11) **EP 1 099 450 B1**
(45) Veröffentlichungstag und Bekanntmachung des Hinweises auf die Patenterteilung: **28.12.2005**
(21) Anmeldenummer: 00123275.0
(22) Anmeldetag: 26.10.2000
(51) Int. Cl.: A61M 5/32

(54) **Schutzkappe für eine Kanüle**
Protection cap for a cannula
Capuchon de protection pour une canule

(30) Priorität: 11.11.1999 DE 19954373
(43) Veröffentlichungstag der Anmeldung: 16.05.2001
(73) Patentinhaber: Helvoet Pharma Belgium N.V., 3570 Alken (BE)
(72) Erfinder: Claessens, Albert Louis Victor Jozef, 3530 Houthalen (BE)
(74) Vertreter: Fehners, Klaus Friedrich

(56) Entgegenhaltungen:
- DE-A- 4 234 319
- US-A- 4 775 367
- US-A- 4 986 818
- US-A- 5 098 400
- US-A- 5 147 325

## Beschreibung

Die Erfindung bezieht sich auf eine Schutzkappe für eine Kanüle, umfassend eine Außenhülse und ein in der Außenhülse angeordnetes Dichtelement, das einen an einem Ende geschlossenen Kanal zur Abdichtung einer Öffnung an der Spitze der Kanüle aufweist, und wobei die Außenhülse an dem hinteren Ende einen im wesentlichen zylindrischen, sich in Richtung des Frontendes hin leicht verjüngenden Sockelabschnitt aufweist, der zum Aufsetzen der Schutzkappe auf einen die Kanüle haltenden Konus der Spritze vorgesehen ist und eine Einführöffnung für die Kanüle aufweist, an den sich ein in seinem Querschnitt in Längsrichtung der Schutzkappe ebenfalls verjüngender Frontabschnitt anschließt, der sich bis zu dem Frontende der Außenhülse erstreckt, und wobei die Außenhülse eine Durchgangsöffnung aufweist, in der das Dichtelement angeordnet ist und die an dem Frontende der Außenhülse durch das Dichtelement verschlossen ist, wobei die Außenhülse aus einer Kunststoff-Hartkomponente und das Dichtelement aus einer gaspermeablen Kunststoff-Weichkomponente hergestellt ist und das Dichtelement und die Außenhülse gemeinsam in einem Zwei-Komponenten-Spritzgußverfahren hergestellt und fest miteinander verbunden sind und zwischen beiden kein Zwischenraum vorhanden ist.

Derartige Schutzkappen sind im medizinischen Bereich anwendbar und für Kanülen sowie mit Kanülen versehene Spritzen bereits seit langem bekannt, um ein steriles Verpacken der Kanülen ohne Beschädigung des Packmittels zu ermöglichen und die Verletzungsrisiken bei der Handhabung zu vermindern. Zudem dienen die Schutzkappen dazu, Beschädigungen an den angeschliffenen, dünnen Spitzen der Kanülen zu verhindern. Bisweilen werden die Schutzkappen auf die benutzten Kanülen auch wieder aufgesetzt.

Vor einer Verwendung müssen die aus der Produktion kommenden Schutzkappen zunächst sterilisiert werden. Genauso sind die Kanülen wie auch die Spritzen zu sterilisieren. Dies erfolgt mit gasförmigen Medien, beispielsweise Dampf oder Äthylenoxydgas (ETO).

Die genannten Elemente werden zur Vermeidung der Übertragung von Krankheiten in der Regel nur einmal verwendet und nach ihrem Gebrauch entsorgt. Angesichts des hohen zahlenmäßigen Bedarfs ist daher eine schnelle und einfache Herstellung von großer Bedeutung. Die Gestaltung dieser Elemente hat jedoch stets im Lichte der Minimierung von Verletzungs- und Infektionsrisiken zu stehen.

Aus der DE 42 34 319 A1 ist eine Schutzkappe für eine Kanüle bekannt, die eine Außenhülse aus einer Kunststoff-Hartkomponente und ein in der Außenhülse vorgesehenes Dichtelement aus einer Kunststoff-Weichkomponente zur Abdichtung einer Öffnung an der Spitze der Kanüle aufweist. Die Herstellung erfolgt in einem Zwei-Komponenten-Spritzgußverfahren. Jedoch ist die bekannte Schutzkappe im Hinblick auf die Sterilisierung sowie auf die Handhabung verbesserungsbedürftig.

Vor der Verwendung wird die bekannte Kanülenschutzkappe sterilisiert. Da die Kunststoff-Hartkomponente der Außenhülse jedoch gasundurchlässig ist, werden die innenliegenden Bereiche nur schwer von einem gasförmigen Sterilisiermedium erreicht, so daß für eine zuverlässige Abtötung aller Keime und Erreger eine lange Verweildauer in einem Sterilisator erforderlich ist. Eine Sterilisation in einem verpackungsfertig aufgesteckten Zustand auf eine Kanüle ist in dieser Hinsicht noch ungünstiger. Überlegungen zur Verringerung der Dauer des Sterilisiervorganges fanden mit Rücksicht auf höchste Reinheitsanforderungen bisher jedoch keinen Raum.

Die Außenhülse aus der Kunststoff-Hartkomponente verleiht der bekannten Schutzkappe nach außen hin eine ausreichende Steifigkeit, während das Dichtelement aus der Kunststoff-Weichkomponente die Öffnung an der Kanülenspitze verschließt. Um das Verletzungs- und Infektionsrisiko gering zu halten, erstreckt sich die Außenhülse durchgehend und ununterbrochen über die gesamte Länge des freiliegenden Bereiches einer zu schützenden Kanüle. Die Ankopplung der Schutzkappe an eine Spritze erfolgt dabei ausschließlich über die Weichkomponente, die an der Einführöffnung allein die Kanülenschutzkappe bildet. Jedoch ist die weiche Ausgestaltung der Einführöffnung der Schutzkappe für ein sicheres Ergreifen beim Wiedereinführen der Kanüle in die Schutzkappe eher unvorteilhaft, da diese dann leicht zu eng zusammengedrückt werden kann.

Der Erfindung liegt demgegenüber die Aufgabe zugrunde, eine Schutzkappe für eine Kanüle zu schaffen, die bei einer effizienten Herstellung und sicheren Handhabung ein exzellentes Sterilisationsverhalten zeigt.

Diese Aufgabe wird durch eine Schutzkappe der eingangs genannten Art gelöst, bei der die Außenhülse zwischen ihrem Frontende und dem Übergangsbereich von dem Sockelabschnitt einerseits und dem Frontabschnitt andererseits an ihrer Längsseite mindestens eine Seitenöffnung aufweist, wobei die mindestens eine Seitenöffnung im Frontabschnitt unmittelbar an den Sockelabschnitt angrenzt und, bezogen auf die Längsrichtung der Schutzkappe, so ausgebildet ist, daß sie sich zumindest in einen Teil des Bereiches des Kanals des Dichtelementes erstreckt, der zur Aufnahme der Kanüle vorgesehen ist, und daß die mindestens eine Seitenöffnung in der Außenhülse durch das Dichtelement abgedeckt ist.

Hierdurch kann eine wirkungsvolle und damit schnelle Sterilisierung der Schutzkappe selbst in einem auf eine Kanüle aufgesetzten Zustand erzielt werden. Der Eindringweg des gasförmigen Sterilisationsmediums in das Innere der Kanülenschutzkappe wird durch die seitlich in deren Außenwand vorgesehenen Öffnungen erheblich verkürzt. Überdies besteht hierdurch keine Notwendigkeit mehr, für den Fall eines gemeinsamen Sterilisierens einer Kanüle und der Schutzkappe in einem auf die Kanüle aufgesetzten Zustand die Einführöffnung der Schutzkappe zur Ermöglichung eines Gasdurchtrittes aus einer Kunststoff-Weichkomponente zu bilden, so daß auch eine die Handhabung verbessernde Konstruktionsweise ermöglicht wird.

In vorteilhafter Ausgestaltung der vorbeschriebenen Schutzkappe erfolgt die Abdeckung der Seitenöffnung in der Außenhülse durch das Dichtungselement in der Weise, daß das Dichtelement in die Seitenöffnung eindringt und diese ausfüllt, und zwar in einer Dicke, die der Dicke der Wandung der Außenhülse entspricht.
Durch diese Ausbildung ergibt sich eine glattflächige, geschlossene Außenform der Schutzkappe. Damit wird nach dem Aufziehen einer Spritze und dem Ansetzen einer sterilen Kanüle mit einer Schutzkappe insbesondere bei einem nicht unmittelbar anschließenden Verspritzen des Spritzeninhaltes jegliche Kontaminationsgefahr für die Kanüle ausgeschlossen.

Nach einer weiteren, vorteilhaften Ausgestaltung der Erfindung ist der Frontabschnitt mit vorzugsweise zwei oder vier jeweils an dem Sockelabschnitt ansetzenden, sich in Längsrichtung der Schutzkappe erstreckenden Rippen versehen.
Diese Ausgestaltung der Schutzkappe stellt eine Sicherung derselben gegen ein Fortrollen im losen Zustand bei gleichzeitig hoher struktureller Steifigkeit dar.

Eine besonders gut zu ergreifende Form ergibt sich dann, wenn die Rippen jeweils gegenüber der Radialrichtung der Außenhülse geneigt angeordnet sind, derart, daß der Frontabschnitt im Querschnitt einer H-förmige Gestalt aufweist. Dabei bieten die breiten, abgeflachten Außenflanken der H-Form eine verhältnismäßig große Anlagefläche für die Finger einer handhabenden Person, die damit die Kappe sicher ergreifen und positionsrichtig zu einer Kanüle führen kann.

Bevorzugt werden dabei die Seitenöffnungen jeweils an Wandabschnitten zwischen zwei im wesentlichen parallelen Rippen angeordnet. Hierdurch wird eine Gestalt geschaffen, die in bezug auf das Verletzungsrisiko ein hohes Sicherheitspotential aufweist, denn der eingezogene Bereich bietet eine hohe Sicherheit für den Fall eines Durchdringens der Schutzkappe im schwächsten Bereich der Außenwand, nämlich an den Seitenöffnungen der Außenhülse.

Eine besonders sichere Lösung ergibt sich in einer weiteren Ausgestaltung der Erfindung, bei der ein die Seitenöffnung in Einschubrichtung einer Kanüle auf das Frontenede hin begrenzender Randabschnitt als nach außen abstehende Schutzzunge ausgebildet ist, deren innenseitiger Wandabschnitt in Richtung des Frontendes der Außenhülse nach innen geneigt ist.
Sollte es aus unerfindlichen Gründen beim Aufsetzen zu einer extremen Schiefstellung der Schutzkappe in Bezug auf die Kanüle kommen, so stößt diese gegen den Wandabschnitt, womit ein Durchdringen der Außenhülse besser verhindert wird. Die Neigung desselben bewirkt eine Ablenkung der schräg eingeführten Kanüle in eine korrekte Lage. Überdies erlaubt die nach außen gebogene Form der Schutzzunge eine größere Ausbildung der Seitenöffnung ohne Sicherheitsverluste, so daß ein großer Eintrittsbereich für das sterilisierende Medium geschaffen werden kann und folglich das Sterilisierverhalten weiter verbessert wird.

In einer weiteren, vorteilhaften Ausgestaltung der Erfindung weist zur weiteren Verbesserung der Handhabung der Schutzkappe der Frontabschnitt mit Bezug auf die Längsachse derselben zwei einander gegenüberliegende Schmalseiten und zwei einander gegenüberliegende Breitseiten auf, wobei letztere jeweils als Greifflächen ausgebildet und mit rutschhemmenden Vorsprüngen und/oder Vertiefungen und/oder Ausnehmungen versehen sind.

Weiterhin kann der Frontabschnitt jeweils mehrere Seitenöffnungen aufweisen, die als längliche Schlitze quer zur Längsrichtung der Schutzkappe ausgebildet sind.
Mit dieser vorteilhaften Ausgestaltung ergibt sich ein hervorragendes Sterilisierverhalten, da die Eindringwege des Sterilisiermediums sehr kurz gehalten werden können. Die schlitzförmige Ausformung der Seitenöffnung bietet nur geringste Angriffsflächen für ein unbeabsichtigtes Durchdringen der Außenwand der Schutzkappe mit einer Kanülenspitze, gewährleistet jedoch einen optimalen Eintritt des Sterilisiermediums. Durch das Ausfüllen der Schlitze mit der Weichkomponente ergibt sich nach außen ein weitestgehend glattflächiges Erscheinungsbild. Ein Verhaken der Schlitze mit umliegenden Gegenständen, das beispielsweise zu einem unbeabsichtigten Abziehen der Schutzkappe von eine Kanüle führen könnte, wird unterbunden. Durch eine noppenartige Vorwölbung des Füllmaterials können optional zudem rutschfeste Greifstellen geschaffen werden.

Die Erfindung wird nachfolgend beispielhaft anhand von in den Zeichnungen dargestellten Ausführungsbeispielen näher erläutert. Die Zeichnungen zeigen in
- Fig. 1: eine räumliche Ansicht eines ersten Ausführungsbeispiels einer Schutzkappe für eine Kanüle;
- Fig. 2: eine Ansicht von oben auf die Schutzkappe von Figur 1;
- Fig. 3: eine Seitenansicht der Schutzkappe von Figur 1, wobei die rechte Hälfte als Schnitt entlang der Linie A-A in Figur 2 dargestellt ist;
- Fig. 4: eine Seitenansicht der Schutzkappe von Figur 1, die gegenüber der Ansicht von Figur 3 um 90° verdreht ist, wobei die rechte Hälfte als Schnitt entlang der Linie B-B in Figur 2 dargestellt ist;
- Fig. 5: eine weitere räumliche Darstellung des ersten Ausführungsbeispiels, bei der ein Viertelsegment entsprechend der Linie X-X in Figur 2 weggeschnitten ist;
- Fig. 6: eine räumliche Darstellung der Schutzkappe nach dem ersten Ausführungsbeispiel in einem auf eine Spritze aufgesetzten Zustand;
- Fig. 7: eine Ansicht von oben auf die Anordnung von Figur 6;
- Fig. 8: eine Ansicht entsprechend Figur 5, zur Veranschaulichung der Lage der Schutzkappe zu einer Spritze mit integrierter Kanüle;
- Fig. 9: eine räumliche Darstellung einer Außenhülse und eines Dichtelementes der Schutzkappe, die hier zur Veranschaulichung nebeneinander dargestellt sind;
- Fig.10: eine räumliche Darstellung entsprechend Figur 9, jedoch mit teilgeschnittener Außenhülse und teilgeschnittenem Dichtelement;
- Fig. 11: ein zweites Ausführungsbeispiel einer Schutzkappe in räumlicher Darstellung, die im Bereich ihrer Seitenöffnungen mit Schutzzungen versehen;
- Fig. 12: die Ansicht von Fig. 11 im teilgeschnittenen Zustand;
- Fig. 13: ein drittes Ausführungsbeispiel einer Schutzkappe in räumlicher Darstellung, die an ihren Greifflächen mit Querrippen versehen ist;
- Fig. 14: eine räumliche Ansicht eines vierten Ausführungsbeispiels einer Schutzkappe für eine Kanüle;
- Fig. 15: eine weitere räumliche Darstellung des vierten Ausführungsbeispiels, bei der ein Viertelsegment der Schutzkappe weggeschnitten ist;
- Fig. 16: eine räumliche Darstellung einer Außenhülse und eines Dichtelementes der Schutzkappe nach dem vierten Ausführungsbeispiel, die hier zur Veranschaulichung nebeneinander dargestellt sind; und in
- Fig. 17: eine räumliche Darstellung entsprechend Figur 16, jedoch mit teilgeschnittener Außenhülse und teilgeschnittenem Dichtelement.

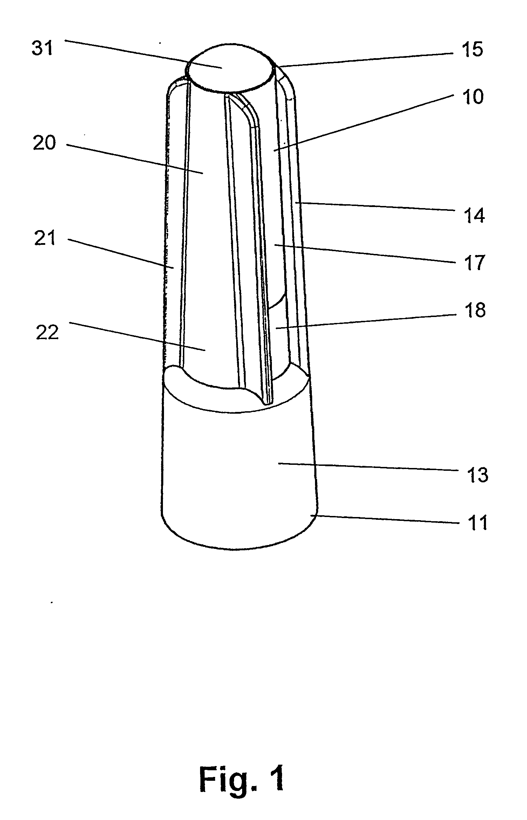

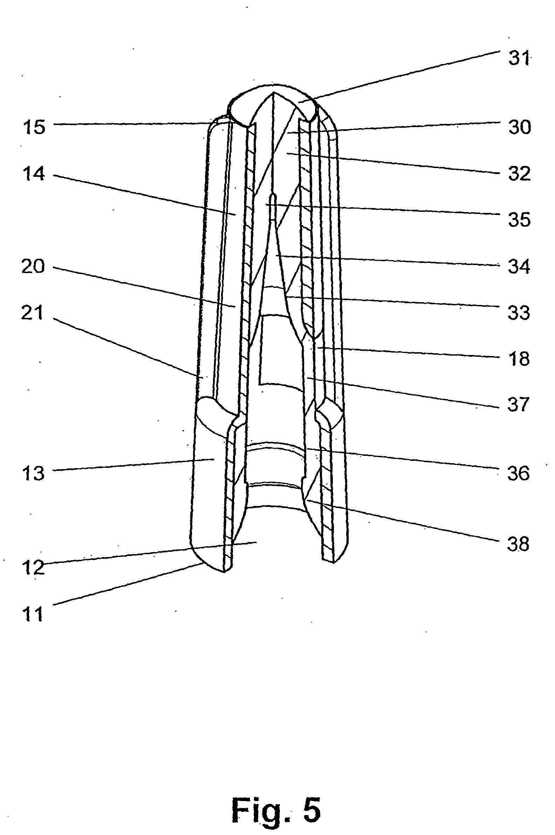

Die Kanülenschutzkappe nach dem ersten Ausführungsbeispiel, die in den Figuren 1 bis 10 dargestellt ist, wird aus einer Außenhülse 10 und einem in der Außenhülse vorgesehenen Dichtelement 30 gebildet. Die Außenhülse 10 verleiht der Schutzkappe eine zum Ergreifen ausreichende Steifigkeit. Überdies ist sie ausreichend fest ausgebildet, um einem Durchstechen mit der Spitze einer Kanüle bzw. Spritzennadel zu widerstehen. Das Dichtelement 30 dient hingegen primär der Abdichtung einer Öffnung an der Spitze der Kanüle sowie einer schützenden, elastischen Lagerung derselben. Damit eignet sich die Schutzkappe besonders zur Konfektionierung mit solchen Spritzen, die gebrauchsfertig montiert und befüllt vertrieben werden. Sie kann jedoch genauso mit separaten Kanülen verwendet werden.

Die Außenhülse 10 der Schutzkappe besteht dabei aus einer Kunststoff-Hartkomponente, wohingegen das Dichtelement 30 aus einer gaspermeablen Kunststoff-Weichkomponente hergestellt ist. Beide sind zusammen in einem Zwei-Komponenten-Spritzgußverfahren hergestellt. Vorzugsweise erfolgt zunächst das Spritzen der Außenhülse 10, an die dann das Dichtelement 30 aus der Weichkomponente angespritzt wird, so daß sich zwischen diesen eine Verbundhaftung einstellt.

Die Außenhülse 10 weist an ihrem breiteren, hinteren Ende 11, das in Längsrichtung eine Einführöffnung 12 für die Kanüle bildet, einen außen im wesentlichen kreiszylindrischen Sockelabschnitt 13 auf, der sich zur besseren Herstellbarkeit in Richtung eines Frontendes 15 der Schutzkappe leicht verjüngt. An den Sockelabschnitt 13 schließt in Einschubrichtung einer Kanüle ein Frontabschnitt 14 an, der sich bis zu dem Frontende 15 der Außenhülse 10 erstreckt. Dabei ist die Außenhülse 10 selbst als Hohlkörper mit einer zentralen Durchgangsöffnung 16 ausgebildet.

Weiterhin ist die Außenhülse 10 an ihrer längsseitigen Außenwand mit mindestens einer Seitenöffnung, hier zwei einander gegenüberliegenden Seitenöffnungen 18 versehen, die an den Sockelabschnitt 13 unmittelbar angrenzend angeordnet sind. In dem ersten Ausführungsbeispiel liegen die Seitenöffnungen 18 bezogen auf die Längsrichtung in etwa in der Mitte der Kanülenschutzkappe. Zweckmäßigerweise sind sie solchermaßen angeordnet, daß sich kurze Eindringwege für ein gasförmiges Sterilisationsmedium, beispielsweise Dampf oder Äthylenoxydgas, ergeben.

Der in seinem Querschnitt unrunde Frontabschnitt 14 weist einen im wesentlichen zylindrischen Grundkörper 20 auf, der sich in dem Ausführungsbeispiel im Hinblick auf eine einfache spritzgußtechnische Herstellung zu dem Frontende 15 hin leicht verjüngt. Dabei ist der Grundkörper 20 mit im wesentlichen gleichmäßiger Wanddicke ausgeführt.

Weiterhin sind an dem Frontabschnitt 14 vier jeweils an dem Sockelabschnitt 13 bündig ansetzende Rippen 21 vorgesehen, die ein Fortrollen der losen Schutzkappe verhindern. Diese Rippen 21 schließen außenseitig an den Grundkörper 20 an und erstrecken sich in Längsrichtung der Schutzkappe. Die Anordnung der Rippen 21 erfolgt jeweils gegenüber der Radialrichtung der Außenhülse derart geneigt, daß der Frontabschnitt 14 im Querschnitt eine H-artige Gestalt aufweist. Der Frontabschnitt 14 weist somit im Bereich der etwa glattwandigen Außenflanken der H-Form zwei einander gegenüberliegende, breite Greifflächen 22 auf, die über die eingezogenen Abschnitte 17 der H-Form, die vergleichsweise schmal ausgebildet sind, miteinander verbunden sind.

Die bereits erwähnten Seitenöffnungen 18 sind jeweils an den eingezogenen Wandabschnitten 17 zwischen zwei im wesentlichen parallelen Rippen 21 angeordnet, so daß selbst dann, wenn eine Kanülenspitze durch die Seitenöffnungen 18 geringfügig nach außen dringen sollte, die Gefahr von Verletzungen der handhabenden Person vermieden wird.

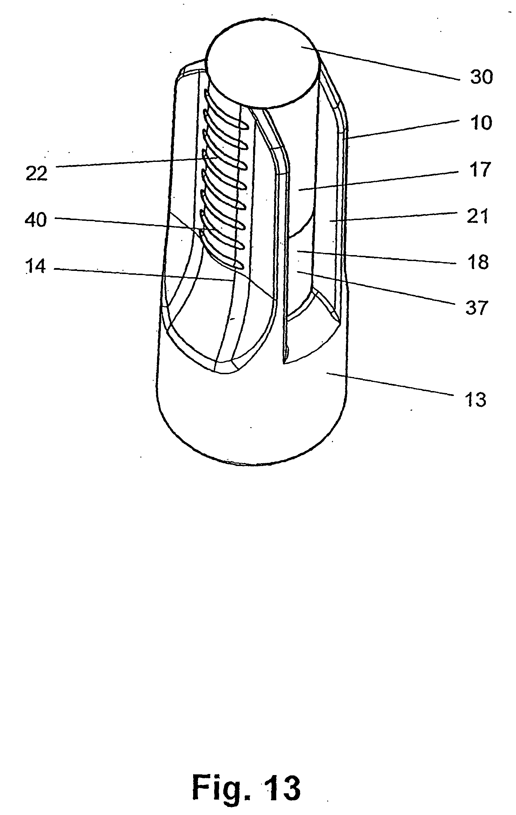

In einer alternativen Ausführungsvariante, die in der Zeichnung nicht dargestellt ist, werden die Seitenöffnungen 18 jeweils durch mehrere, quer zur Längsrichtung verlaufende Schlitze gebildet, die in bezug auf die Längsrichtung in Reihe angeordnet sind, wie dies in Figur 13 für die rutschhemmenden Vorsprünge bzw. Vertiefungen 40 dargestellt ist. Die Schlitze liegen dabei im wesentlichen im Bereich der in den Figuren 1 bis 10 dargestellten Öffnungen 18. Hierdurch kann mit noch größerer Sicherheit ein Herausdringen der Kanüle an dieser Stelle verhindert werden.

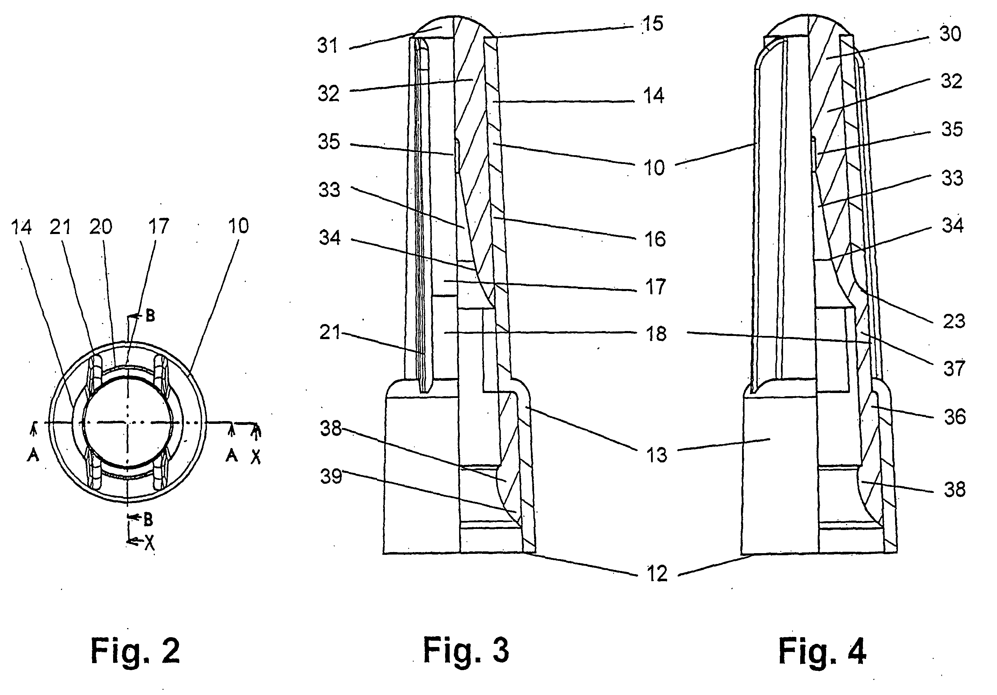

Wie insbesondere aus den Figuren 4 bis 10 weiter zu erkennen ist, sitzt das Dichtelement 30 innerhalb der Außenhülse 10. Lediglich ein pilzartiger Frontendabschnitt 31 ragt durch eine Axialöffnung 19 am Frontende 15 der Außenhülse 10 etwas hervor, um die Kanülenschutzkappe mit einer weichen Spitze und leicht gekrümmt abzuschließen.

Hinter dem Frontendabschnitt 31 des Dichtelementes 30 weist letzteres einen Dichtstopfenabschnitt 32 auf, in dem innenseitig ein einseitig geschlossener Aufnahmekanal 33 für die Kanüle ausgebildet ist. Der geschlossene Kanal 33 des Dichtelementes 30 ist zu seinem geschlossenen Ende hin zunächst mit einem sich kontinuierlich verjüngenden Abschnitt 34 ausgebildet, dessen Querschnitt anfänglich ein Mehrfaches des Durchmessers des röhrenartigen Abschnittes einer Kanüle beträgt. Der Abschnitt 34 geht dann in einen Endabschnitt 35 des Kanals 33 über, in den die Öffnung an der Spitze der Kanüle im voll eingeschobenen Zustand eindringt. Der Innendurchmesser des Endabschnittes 35 ist dabei kleiner, als der Außendurchmesser des Frontabschnittes der Kanüle, so daß das elastische Material des Dichtelementes etwas verformt wird. Durch diese Vorformung des als Sackloch ausgebildeten, auf der Längsachse der Kanülenschutzkappe angeordneten Spitzenaufnahmekanals 33 bleiben der Einschubwiderstand und auch die auf die Kanülenspitze bei nicht exakter Führung gegebenenfalls einwirkenden Biegekräfte gering.

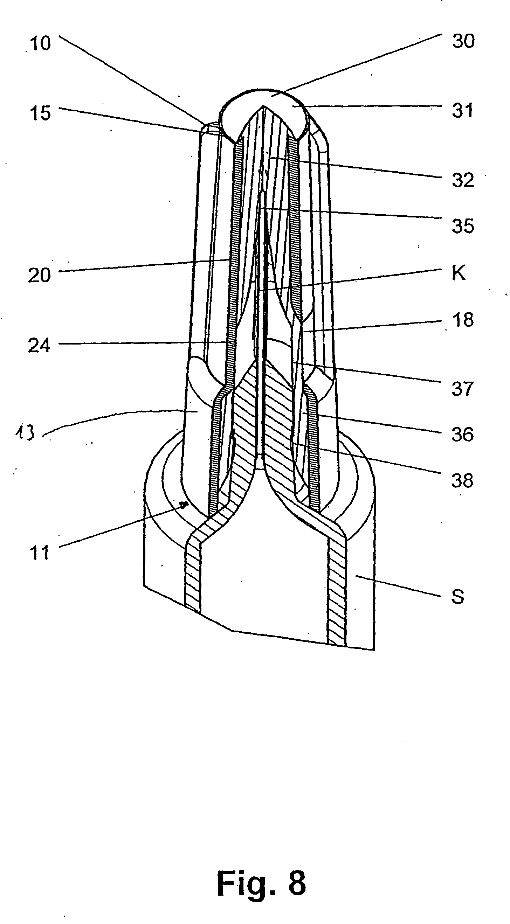

An den Dichtstopfenabschnitt 32 schließt, wie in Figur 8 zu erkennen ist, in Richtung auf die Einführöffnung 12 ein innenseitig leicht konischer Abschnitt 36 an, gegen den ein Konus einer Kanüle oder ein Sockel einer Spritze im eingeschobenen Zustand zur Anlage gebracht werden kann. Der konische Abschnitt 36 kann zwar mit unterschiedlichen Kanülen- bzw. Spritzentypen verwendet werden, es empfiehlt sich jedoch eine Formabstimmung auf die verwendeten Kanülen bzw. Spritzen.

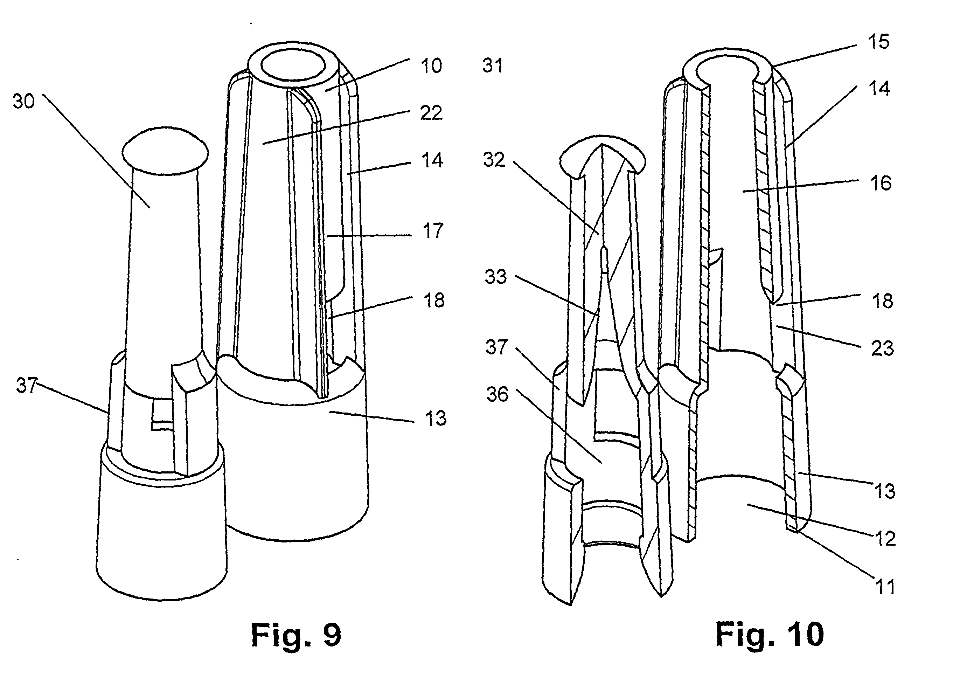

Der leicht konische Abschnitt 36 deckt weiterhin die Seitenöffnungen 18 der Außenhülse 10 ab. Zudem ist der Frontabschnitt 14 an seiner Innenwand im Bereich der Seitenöffnungen 18 jeweils mit einer sich in Längsrichtung der Außenhülse 10 erstreckenden Nut 23 versehen, die an ihrem sockelseitigen Ende offen ist. Wie aus den Figuren 9 und 10 zu erkennen ist, weist das Dichtelement 30 an der Außenseite des leicht konischen Abschnittes 36 in die Nuten 23 eingreifende, diese vorzugsweise ausfüllende Radialvorsprünge 37 auf.

Das Dichtelement 30 erstreckt sich vom Dichtstopfenabschnitt 32 in Richtung auf die Einführöffnung 12 relativ dünnwandig entlang der Innenwand zunächst des Frontabschnittes 14 und dann des Sockelabschnittes 13 der Außenhülse 10. Wie aus den Figuren 9 und 10 zu erkennen ist, bedeckt das Dichtelement 30 die Innenwand der Außenhülse 10 nicht vollständig. Vielmehr bleiben im Bereich des leicht konischen Abschnittes 36 auf Höhe der Seitenöffnungen 18 Abschnitte 24 der Innenwand der Außenhülse 10 unbedeckt. Jedoch erstreckt sich das Dichtelement 30, wie bereits erwähnt, über die Seitenöffnungen 18, um die Kanüle gegen eine Kontamination durch das Eindringen von Fremdpartikeln von außen zu schützen.

Das hintere Ende des Dichtelementes 30 wird durch einen den freien Innendurchmesser der Schutzkappe verengenden Dichtvorsprung mit einer Dichtlippe 38 gebildet, der in einem sich erweiternden Ende 39 ausläuft.

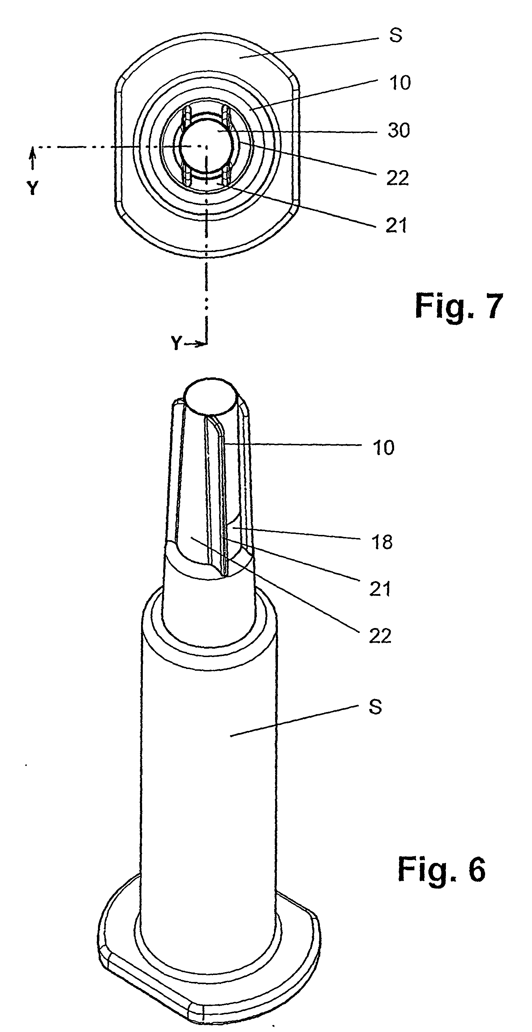

Das rückseitige Ende der Kanülenschutzkappe wird durch die Außenhülse 10 gebildet, die aus der Kunststoff-Hartkomponente besteht, so daß sich ein grifffester Rand am hinteren Ende der Schutzkappe ergibt. Gleichzeitig bildet der hintere Rand der Außenhülse 10 einen Anschlag gegen ein zu weites Aufstecken der Schutzkappe auf die Kanüle. Im vollständig aufgeschobenen Zustand der Schutzkappe, der in den Figuren 6 bis 8 dargestellt ist, liegt die Spritze bzw. Kanüle in Radialrichtung lediglich gegen die elastische Weichkomponente an, während die Axialabstützung über die Hartkomponente erfolgt.

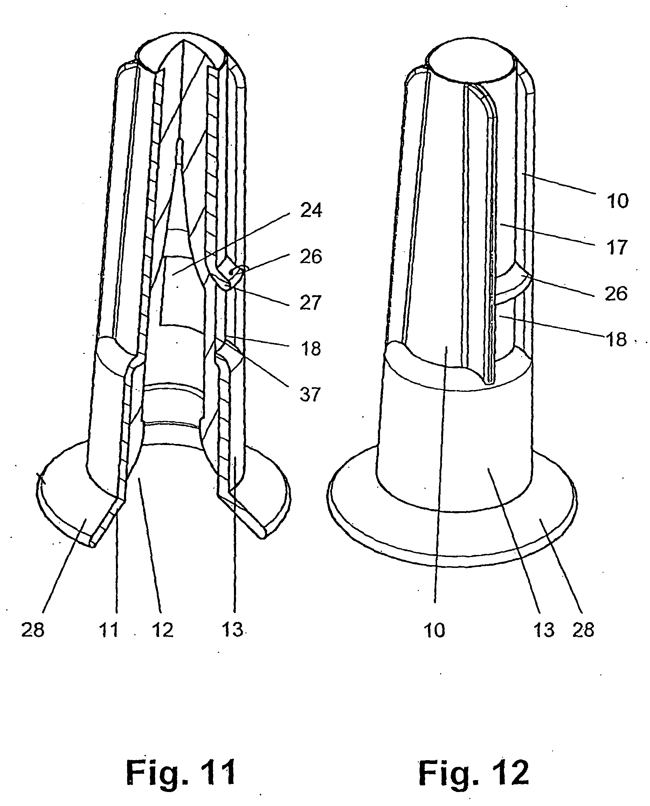

Das zweite Ausführungsbeispiel in den Figuren 11 und 12 stellt eine Abwandlung des ersten Ausführungsbeispiels dar, so daß im folgenden nur noch die unterschiedlichen Merkmale erläutert werden.

Eine Besonderheit des zweiten Ausführungsbeispiels liegt in der Ausbildung des in Einschubrichtung einer Kanüle vorne liegenden Randbereiches der Seitenöffnungen 18. Dieser Randbereich ist hier als nach außen abstehende Schutzzunge 26 ausgebildet, welche die Wand des eingezogenen Bereiches 17 in Richtung auf die Einführöffnung 12 nach außen geneigt oder gekrümmt fortsetzt. Das axiale Ende der Schutzzunge 26 bleibt dabei jedoch innerhalb der zwischen den jeweiligen Rippen 21 gebildeten Nut. Die durch die Schutzzunge 26 innenwandseitig gebildete Schrägung 27 verhindert selbst bei einer extremen Schrägstellung einer Kanüle deren Herausdringen aus der Außenhülse 10 und bewirkt gegebenenfalls eine Korrektur der Kanülenspitze zum Inneren der Schutzkappe hin. Die Anbringung der Schutzzunge 26 ermöglicht einen größeren Anordnungsbereich der Seitenöffnungen 18 oder einen größeren Öffnungsquerschnitt derselben, wie nachfolgend kurz erlautert wird.

Die maximale Schrägstellung einer Kanüle wird praktisch durch einen Spritzensockel bzw. einen Konus um die Kanüle begrenzt, der bei einer Schrägstellung irgendwann gegen Abschnitte der Schutzkappe anstößt, wodurch ein falsches Aufsetzen unter extremen Winkeln vermieden wird. Befindet sich eine Seitenöffnung 18 sehr nahe an der Einführöffnung 12 der Schutzkappe, sind mit der Kanüle noch relativ starke Schrägstellungen möglich bis eine Anschlagwirkung eintritt. Der Sockelabschnitt 13 der Außenhülse 10 ist daher für die Positionierung der Seitenöffnungen 18 weniger geeignet. Mit zunehmender Einschubtiefe nimmt die maximal mögliche Schrägstellung der Kanüle ab. Bereits durch eine geringe Verlegung des frontseitigen Randes der Seitenöffnungen 18 nach außen kann ein erheblicher Längengewinn der Seitenöffnungen 18 in Längsrichtung erzielt werden, in dem die Kanüle die Seitenöffnungen 18 nicht mehr erreichen kann. Die in der Figur 12 gezeigte Position der Seitenöffnungen 18 am Ende des Sockelabschnittes 13 und in etwa in der Mitte der Außenhülse 10 ist in dieser Hinsicht sowie unter Berücksichtigung einer schnellen Sterilisierbarkeit als besonders günstig anzusehen.

Eine weitere Besonderheit des zweiten Ausführungsbeispiels liegt in der Ausgestaltung des rückseitigen Randes der Schutzkappe, der zu einem sichereren Einführen der Kanüle in die Außenhülse 10 einen sich in Radialrichtung erstreckenden Kragen 28 als Fingerschutz aufweist. Dieser Kragen 28 ist leicht trichterartig bzw. konisch ausgebildet, um bei einem Verfehlen der Einführöffnung 12 ein Abgleiten der Kanüle nach außen in den Greifbereich der Finger einer handhabenden Person zu verhindern. Die trichterartige Form korrigiert vielmehr eine Fehlführung in Richtung auf die Einführöffnung 12 hin.

Ein drittes Ausführungsbeispiel ist in Figur 13 dargestellt, das entsprechend dem ersten Ausführungsbeispiel ausgebildet ist. Entsprechend dem zweiten Ausführungsbeispiel kann jedoch auch die Schutzkappe nach Figur 13 mit Schutzzungen im Bereich der Seitenöffnungen 18 versehen werden. Das dritte Ausführungsbeispiel unterscheidet sich von den zuvor beschriebenen Ausführungsbeispielen durch das Vorhandensein von Querrippen 40, die an den Greifflächen 22 des Frontabschnittes 14 für ein rutschfreies Erfassen der Schutzkappe vorgesehen sind. Anstelle der Rippen können auch Quernuten, Noppen oder dergleichen an der Außenhülse 10 vorgesehen werden. In einer weiteren Variante werden die Rippen 40 durch Material der Weichkomponente gebildet, das außenseitig angespritzt ist, oder aber mit dem Dichtelement 30 verbunden ist und sich durch entsprechende Querschlitze der Außenhülse 10 nach außen erstreckt.

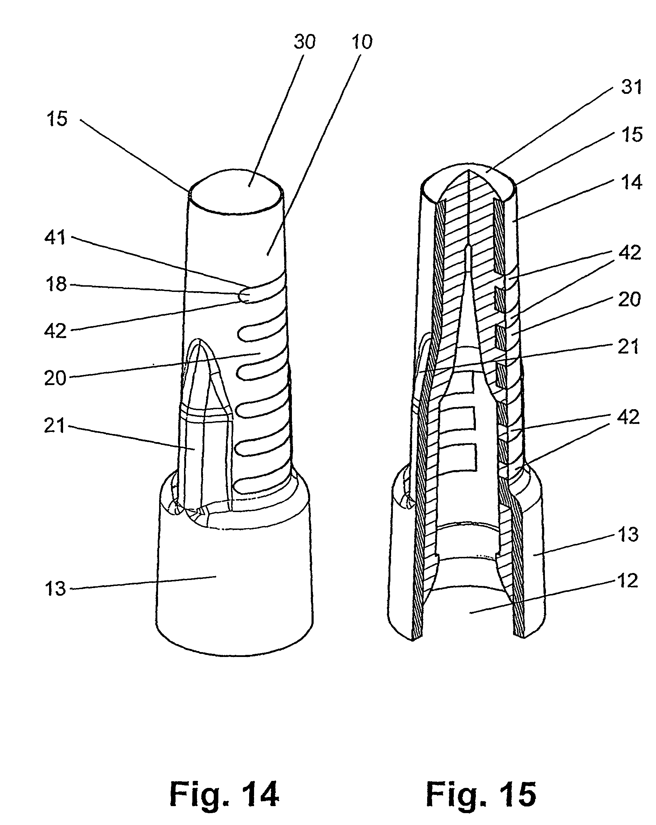

Im folgenden wird nun anhand der Figuren 14 bis 17 ein viertes Ausführungsbeispiel einer Schutzkappe für eine Kanüle beschrieben. Dieses unterscheidet sich von dem ersten Ausführungsbeispiel vor allem durch die Ausbildung der Außenhülse 10 im Hinblick auf die Ausbildung und Anordnung der Seitenöffnungen 18 sowie die Ausgestaltung der Rippen 21 an dem Grundkörper 20.

Die Schutzkappe des vierten Ausführungsbeispiels umfaßt wie das erste Ausführungsbeispiel eine Außenhülse 10 aus einer Kunststoff-Hartkomponente sowie ein in der Außenhülse angeordnetes Dichtelement 30 aus einer Kunststoff-Weichkomponente.

Wie insbesondere aus den Figuren 14, 16 und 17 zu erkennen ist, weist die Außenhülse 10 einen Sockelabschnitt 13 auf, an den ein Frontabschnitt 14 unmittelbar anschließt. Der Frontabschnitt 14 umfaßt einen sich konisch zu einem Frontende 15 verjüngenden, zylindrischen Grundkörper 20, der als Hülse mit einer Durchgangsöffnung 16 ausgebildet ist. Die im wesentlichen konische Außenform des Grundkörpers 20 wird durch zwei sich in Längsrichtung der Schutzkappe erstreckende Rippen 21 unterbrochen, die unmittelbar an dem Sockelabschnitt 13 ansetzen. In dem dargestellten Ausführungsbeispiel sind die Rippen 21 einander gegenüberliegend angeordnet und enden in etwa in der Mitte des Frontabschnittes 14, so daß zwischen zwei benachbarten Rippen 21 quasi ein eingezogener Abschnitt 17 gebildet wird, an dem die Seitenöffnungen 18 vorgesehen sind. Die Rippen 21 setzen hingegen die größere Außenweite des Sockelabschnittes 13 in den Bereich des Frontabschnittes 14 fort. Neben einer verbreiterten Greifmöglichkeit verhindern die Rippen 21 ein Fortrollen der Schutzkappe im von einer Spritze gelösten Zustand.

Aus Figur 14 ist zu entnehmen, daß zwischen zwei benachbarten Rippen 21 sowie auch in Längsrichtung der Schutzkappe über die Rippen 21 hinaus jeweils mehrere Seitenöffnungen 18 vorgesehen sind, die hier als im wesentlichen parallel zueinander verlaufende, längliche Schlitze 41 quer zur Längsrichtung der Schutzkappe ausgebildet sind. Die Anordnung einer Vielzahl von Schlitzen 41 ermöglicht kurze Eindringwege für ein Sterilisiermedium. Aufgrund der in Längsrichtung der Schutzkappe geringfügigen Breite der Schlitze 41 wird ein Eindringen der Spitze einer Kanüle in einen Schlitz bzw. ein Nachaußendringen durch einen solchen verhindert.

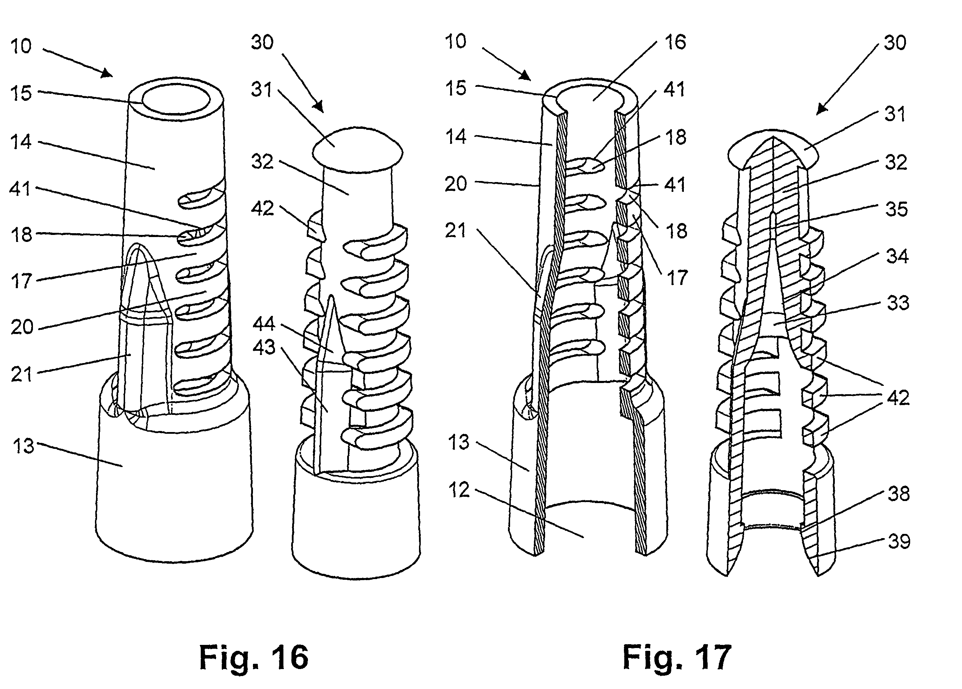

Die Schlitze 41 der Außenhülse 10 werden durch Material der Weichkomponente ausgefüllt, wodurch sich an dem Dichtelement 30 entsprechende, rippenartige Ansätze 42 ergeben. In Figur 15 schließen die rippenartigen Ansätze 42 glattflächig mit der Außenwand des Grundkörpers 20 ab. Allerdings ist es auch möglich, die Ansätze 42 über die Außenwand des Grundkörpers 20 etwas erhaben auszubilden, wodurch eine Riffelung entsteht, welche die Griffigkeit der Schutzkappe im Bereich zwischen den Rippen 21 erhöht. Wie insbesondere aus Figur 16 zu erkennen ist, weist das Dichtelement 30 zwei rippenartige Vorsprünge 43 mit zu dem Frontende 15 hin abgeschrägten Enden 44 auf, die im Bereich der Rippen 21 der Außenhülse 10 angeordnet sind, und in entsprechende Ausnehmungen an der Innenwand der Außenhülse 10 eingreifen.

Im übrigen ist das Dichtelement 30 des vierten Ausführungsbeispieles entsprechend dem Dichtelement 30 des ersten Ausführungsbeispieles ausgebildet, was insbesondere die Innenform mit dem an einem Ende geschlossenen Kanal 33 zur Abdichtung einer Öffnung an der Spitze einer Kanüle betrifft.

## Patentansprüche

1. Schutzkappe für eine Kanüle, umfassend eine Außenhülse (10) und ein in der Außenhülse (10) angeordnetes Dichtelement (30), das einen an einem Ende geschlossenen Kanal (33) zur Abdichtung einer Öffnung an der Spitze der Kanüle aufweist, und wobei die Außenhülse (10) an dem hinteren Ende (11) einen im wesentlichen zylindrischen, sich in Richtung des Frontendes (15) hin leicht verjüngenden Sockelabschnitt (13) aufweist, der zum Aufsetzen der Schutzkappe auf einen die Kanüle haltenden Konus der Spritze vorgesehen ist und eine Einführöffnung (12) für die Kanüle aufweist, an den sich ein in seinem Querschnitt in Längsrichtung der Schutzkappe ebenfalls verjüngender Frontabschnitt (14) anschließt, der sich bis zu dem Frontende (15) der Außenhülse erstreckt, und wobei die Außenhülse (10) eine Durchgangsöffnung (16) aufweist, in der das Dichtelement (30) angeordnet ist und die an dem Frontende (15) der Außenhülse (10) durch das Dichtelement (30) verschlossen ist, wobei die Außenhülse (10) aus einer Kunststoff-Hartkomponente und das Dichtelement (30) aus einer gaspermeablen Kunststoff-Weichkomponente hergestellt ist und das Dichtelement (30) und die Außenhülse (10) gemeinsam in einem Zwei-Komponenten-Spritzgußverfahren hergestellt und fest miteinander verbunden sind und zwischen beiden kein Zwischenraum vorhanden ist, **dadurch gekennzeichnet, daß** die Außenhülse (10) zwischen ihrem Frontende (15) und dem Übergangsbereich von dem Sockelabschnitt (13) einerseits und dem Frontabschnitt (14) andererseits an ihrer Längsseite mindestens eine Seitenöffnung (18) aufweist, wobei die mindestens eine Seitenöffnung (18) im Frontabschnitt (14) unmittelbar an den Sockelabschnitt (13) angrenzt und, bezogen auf die Längsrichtung der Schutzkappe, so ausgebildet ist, daß sie sich zumindest in einen Teil des Bereiches des Kanals (33) des Dichtelementes (30) erstreckt, der zur Aufnahme der Kanüle vorgesehen ist, und daß die mindestens eine Seitenöffnung (18) in der Außenhülse (10) durch das Dichtelement (30) abgedeckt ist.

2. Schutzkappe nach Anspruch 1, **dadurch gekennzeichnet, daß** die Abdeckung der Seitenöffnung (18) in der Außenhülse (10) durch das Dichtungselement (30) in der Weise erfolgt, daß das Dichtelement (30) in die Seitenöffnung (18) eindringt und ausfüllt, und zwar in einer Dicke, die der Dicke der Wandung der Außenhülse (10) entspricht.

3. Schutzkappe nach einem der Ansprüche 1 oder 2, **dadurch gekennzeichnet, daß** der Frontabschnitt (14) mit vorzugsweise zwei oder vier jeweils an dem Sockelabschnitt (13) ansetzenden, sich in Längsrichtung der Schutzkappe erstreckenden Rippen (21) versehen ist.

4. Schutzkappe nach Anspruch 3, **dadurch gekennzeichnet, daß** die Rippen (21) jeweils gegenüber der Radialrichtung der Außenhülse (10) geneigt angeordnet sind, derart, daß der Frontabschnitt (14) im Querschnitt eine H-förmige Gestalt aufweist.

5. Schutzkappe nach Anspruch 4, **dadurch gekennzeichnet, daß** die Seitenöffnung (18) an Wandabschnitten (17) zwischen zwei im wesentlichen parallelen Rippen (21) angeordnet ist.

6. Schutzkappe nach einem der Ansprüche 1 bis 5, **dadurch gekennzeichnet, daß** ein die Seitenöffnung (18) in Einschubrichtung einer Kanüle auf das Frontende (15) hin begrenzender Randabschnitt als nach außen abstehende Schutzzunge (26) ausgebildet ist, deren innenseitiger Wandabschnitt (27) in Richtung des Frontendes (15) nach innen geneigt ist.

7. Schutzkappe nach Anspruch 4, **dadurch gekennzeichnet, daß** der Frontabschnitt (14) mit Bezug auf die Längsrichtung der Schutzkappe zwei einander gegenüberliegende Schmalseiten und zwei einander gegenüberliegende Breitseiten aufweist, wobei letztere jeweils als Greifflächen (22) ausgebildet und mit rutschhemmenden Vorsprüngen und/oder Vertiefungen und/oder Ausnehmungen (40) versehen sind.

8. Schutzkappe nach Anspruch 3, **dadurch gekennzeichnet, daß** der Frontabschnitt (14) jeweils mehrere Seitenöffnungen (18) zwischen den benachbarten Rippen (21) aufweist, die als längliche Schlitze (41) quer zur Längsrichtung der Schutzkappe ausgebildet sind.

## Claims

1. Safety cap for a cannula, comprising an outer sleeve (10) and a seal element (30) arranged inside the outer sleeve (10), said seal element (30) comprising a channel (33), which is closed at one end, for sealing an opening at the tip of the cannula, and wherein the outer sleeve (10) comprises, at the rear end (11), a substantially cylindrical base section (13), which tapers slightly towards the front end (15) and which is provided for placing the safety cap onto a cone of the syringe, said cone holding the cannula, said base section (13) comprising an insertion opening (12) for the cannula and having connected to it a front section (14), which also tapers in its cross section along the longitudinal direction of the safety cap and which extends as far as the front end (15) of the outer sleeve, and wherein the outer sleeve (10) comprises a pass-through opening (16), in which the seal element (30) is arranged and which is closed at the front end (15) of the outer sleeve (10) by the seal element (30), said outer sleeve (10) being made of a hard plastic component and said seal element (30) being made of a gas-permeable soft plastic component, and said seal element (30) and said outer sleeve (10) being produced together in a two-component injection moulding process and being permanently connected to each other with no gap being present between them, **characterised in that**
said outer sleeve (10) comprises, between its front end (15) and the passage from the base section (13), on the one hand, to the front section (14), on the other hand, at least one lateral opening (18) at the long side thereof, wherein the at least one lateral opening (18) is directly adjacent to the base section (13) in the front section (14) and is provided such, in relation to the longitudinal direction of the safety cap, that it extends at least into a part of the area of the channel (33) of the seal element (30) which is provided for reception of the cannula, and that the at least one lateral opening (18) is covered by the seal element (30) in the outer sleeve (10).

2. Safety cap according to claim 1, **characterised in that** the lateral opening (18) in the outer sleeve (10) is covered by the seal element (30) in such a way that the seal element (30) penetrates into the lateral opening (18) and fills it to a thickness which corresponds to the thickness of the wall of the outer sleeve (10).

3. Safety cap according to any one of claims 1 or 2, **characterised in that** the front section (14) is provided with preferably two or four ribs (21), each of which begins at the base section (13) and extends in the longitudinal direction of the safety cap.

4. Safety cap according to claim 3, **characterised in that** the ribs (21) are each arranged at a slant to the radial direction of the outer sleeve (10) in such a way that the front section (14) has the shape of an H in cross section.

5. Safety cap according to claim 4, **characterised in that** the lateral opening (18) is located at wall sections (17) between to essentially parallel ribs (21).

6. Safety cap according to any one of claims 1 to 5, **characterised in that** an edge section limiting the lateral opening (18) in the direction of insertion of the cannula towards the front end (15) is provided as an outwardly protruding safety tongue (26) having an interior wall section (27) which slants inward towards the front end (15).

7. Safety cap according to claim 4, **characterised in that** the front section (14) has, in reference to the longitudinal direction of the safety cap, two opposing narrow sides and two opposing wide sides, the latter being designed as gripping surfaces (22) provided with anti-slip projections and/or depressions and/or recesses (40).

8. Safety cap according to claim 3, **characterised in that** the front section (14) respectively comprises several lateral openings (18) between the adjacent ribs (21), said lateral openings (18) being provided as elongated slots (41) transverse to the longitudinal direction of the safety cap.

## Revendications

1. Capuchon de protection d'une canule, comprenant une douille extérieure (10) et un élément d'étanchéité (30) disposé dans la douille extérieure (10), lequel comporte un canal (33) fermé à une extrémité pour rendre étanche une ouverture à la pointe de la canule, et dans lequel la douille extérieure (10) comporte, à l'extrémité arrière (11), une section de socle (13) sensiblement cylindrique, se rétrécissant légèrement en direction de l'extrémité frontale (15), qui est prévue pour placer le capuchon de protection sur un cône de la seringue, maintenant la canule, et qui présente une ouverture d'introduction (12) de la canule, à laquelle fait suite un segment frontal (14) se rétrécissant également dans sa section transversale dans la direction longitudinale du capuchon de protection et qui s'étend jusqu'à l'extrémité frontale (15) de la douille extérieure, et dans lequel la douille extérieure (10) présente une ouverture de passage (16) dans laquelle est disposé l'élément d'étanchéité (30) et qui est fermée à l'extrémité frontale (15) de la douille extérieure (10) par l'élément d'étanchéité (30), dans lequel la douille extérieure (10) est fabriquée dans un composant dur en matière plastique et l'élément d'étanchéité (30) est fabriqué dans un composant souple en matière plastique perméable aux gaz, et l'élément d'étanchéité (30) ainsi que la douille extérieure (10) sont fabriqués ensemble par un procédé de moulage par injection de deux composants et sont assemblés entre eux de manière fixe et aucun interstice ne se trouve entre eux deux, **caractérisé en ce que** la douille extérieure (10) présente au moins une ouverture latérale (18) entre son extrémité frontale (15) et la zone de transition entre la section de socle (13) d'une part et le segment frontal (14) d'autre part, sur son côté longitudinal, la ou les ouvertures latérales (18) du segment frontal (14) étant directement adjacentes à la section de socle (13) et étant réalisées, par rapport à la direction longitudinale du capuchon de protection, de manière à s'étendre au moins dans une partie de la zone du canal (33) de l'élément d'étanchéité (30) qui est prévue pour recevoir la canule, et **en ce que** la ou les ouvertures latérales (18) de la douille extérieure (10) sont recouvertes par l'élément d'étanchéité (30).

2. Capuchon de protection selon la revendication 1, **caractérisé en ce que** le recouvrement de l'ouverture latérale (18) de la douille extérieure (10) s'effectue par l'élément d'étanchéité (30) de manière que l'élément d'étanchéité (30) pénètre à l'intérieur de l'ouverture latérale (18) et la remplisse, et ce sur une épaisseur qui correspond à l'épaisseur de la paroi de la douille extérieure (10).

3. Capuchon de protection selon l'une des revendications 1 ou 2, **caractérisé en ce que** le segment frontal (14) est pourvu de préférence de deux ou quatre nervures (21) commençant chacune sur la section de socle (30) et s'étendant dans la direction longitudinale du capuchon de protection.

4. Capuchon de protection selon la revendication 3, **caractérisé en ce que** les nervures (21) sont disposées chacune inclinées par rapport à la direction radiale de la douille extérieure (10), de manière que le segment frontal (14) présente dans sa section transversale une forme en H.

5. Capuchon de protection selon la revendication 4, **caractérisé en ce que** l'ouverture latérale (18) est disposée sur des segments de paroi (17) entre deux nervures (21) sensiblement parallèles.

6. Capuchon de protection selon l'une des revendications 1 à 5, **caractérisé en ce qu'**un segment de bordure, délimitant l'ouverture latérale (18) dans le sens d'introduction d'une canule, vers l'extrémité frontale (15), est réalisé sous la forme d'une languette de protection (26) faisant saillie vers l'extérieur dont le segment de paroi (27) côté intérieur est incliné vers l'intérieur en direction de l'extrémité frontale (15).

7. Capuchon de protection selon la revendication 4, **caractérisé en ce que** le segment frontal (14) présente, par rapport à la direction longitudinale du capuchon de protection, deux petits côtés se faisant face l'un l'autre et deux grands côtés se faisant face l'un l'autre, chacun de ces derniers étant réalisés en tant que surfaces de prise (22) et étant pourvus de saillies empêchant le glissement et/ou de creux et/ou d'évidements (40).

8. Capuchon de protection selon la revendication 3, **caractérisé en ce que** le segment frontal (14) présente plusieurs ouvertures latérales (18) entre les nervures (21) voisines, qui sont réalisées sous la forme de fentes (41) allongées, transversalement à la direction longitudinale du capuchon de protection.
